(19) Europäisches Patentamt
European Patent Office
Office européen des brevets

(11) Veröffentlichungsnummer : **0 141 200**
**B1**

(12) # EUROPÄISCHE PATENTSCHRIFT

(45) Veröffentlichungstag der Patentschrift : **27.07.88**

(21) Anmeldenummer : **84110988.7**

(22) Anmeldetag : **14.09.84**

(51) Int. Cl.⁴ : **C 07 F 9/38**// G01N33/20, A61K7/16, A61K31/66

(54) **Dihydroxyalkandiphosphonsäuren.**

(30) Priorität : **22.09.83 DE 3334211**

(43) Veröffentlichungstag der Anmeldung : **15.05.85 Patentblatt 85/20**

(45) Bekanntmachung des Hinweises auf die Patenterteilung : **27.07.88 Patentblatt 88/30**

(84) Benannte Vertragsstaaten : **AT BE CH DE FR GB IT LI LU NL SE**

(56) Entgegenhaltungen :
**EP-A- 0 085 321**
**US-A- 3 733 270**
**US-A- 3 983 227**
**Die Akte enthält technische Angaben, die nach dem Eingang der Anmeldung eingereicht wurden und die nicht in dieser Patentschrift enthalten sind.**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Blum, Helmut**
**Bertha-van-Suttner-Strasse 30**
**D-4000 Düsseldorf 13 (DE)**

**EP 0 141 200 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind α,ω-Dihydroxyalkan-α,α-diphosphonsäuren der allgemeinen Formel (I) :

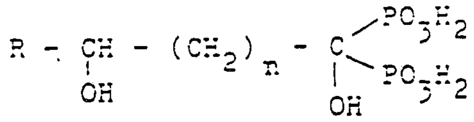

(I)

worin R ein Wasserstoffatom, einen Phenylrest oder einen durch Halogen, Alkylreste oder Hydroxylgruppen substituierten Phenylrest und n eine ganze Zahl im Bereich von 1 bis 9 bedeuten, deren wasserlösliche Salze sowie deren Verwendung als Komplexiermittel gegenüber zwei- und mehrwertigen Metallionen.

Gegenstand der US-A-3 733 270 ist ein Verfahren zur Inhibierung der Ausfällung schwerlöslicher Metallsalze in wäßrigen Systemen mit Hilfe von substituierten Ethan-diphosphonsäuren bzw. deren Salzen. Als inhibitoren werden hier unter anderem auch die 1,2-Dihydroxyethan-1,1-diphosphonsäure und deren Salze genannt. Man gewinnt derartige substituierte Ethan-diphosphonsäuren ausgehend von der Ethylen-diphosphonsäure durch Oxidation mit Wasserstoffperoxid und anschließender Ringöffnungsreaktion der primär gebildeten Epoxyethan-diphosphonsäure. Wird diese Ringöffnung mit Wasser durchgeführt, so resultiert die oben genannte 1,2-Dihydroxyethan-1,1-diphosphonsäure.

Die Herstellung der neuen Diphosphonsäuren erfolgt durch Umsetzung von ω-Amino-α-hydroxyalkan-α,α-diphosphonsäuren mit Salpetriger Säure. Im allgemeinen wird die Amino-phosphonsäure in wäßriger Lösung beziehungsweise Suspension vorgelegt und langsam beziehungsweise portionsweise, d. h. im Verlauf mehrerer Stunden, beispielsweise im Verlauf von 10 bis 15 Stunden, mit Salpetriger Säure versetzt. Die Salpetrige Säure wird vorzugsweise in Form einer wäßrigen Natriumnitritlösung eingesetzt, wobei sich die eigentliche Säure im Verlauf der Reaktion intermediär bildet. Die Zugabe einer weiteren Säure zum Reaktionsgemisch ist im allgemeinen nicht erforderlich, da sich der zur Bildung von Salpetriger Säure erforderliche pH-Bereich durch die vorgelegte Diphosphonsäure ohnehin einstellt.

Anstelle von Natriumnitrit können auch andere Stoffe, die während der Reaktion Salpetrige Säure bilden, wie beispielsweise $N_2O_3$ oder NOCl Verwendung finden. Im Falle solch gasförmiger Reaktionspartner werden diese durch Einleiten in die Suspension eingebracht.

Während der Umsetzung werden im Reaktionsgemisch Temperaturen, die oberhalb der Raumtemperatur liegen, eingehalten ; vorzugweise Temperaturen im Bereich von 40-60 °C, insbesondere bei ca. 50 °C.

Im Interesse einer vollständigen Umsetzung der eingesetzten Aminoalkandiphosphonsäuren zu den gewünschten Endprodukten hat sich ein Überschuß an Salpetriger Säure — beziehungsweise an Natriumnitrit — in der 3-4 fachen molaren Menge, bezogen auf das Ausgangsprodukt, als vorteilhaft erwiesen. Jedoch ist einerseits eine weitere Steigerung dieses Überschusses durchaus möglich, andererseits führt auch ein geringerer Überschuß zu den gewünschten Endprodukten. Das langsame Hinzufügen der Reaktionskomponenten « $HNO_2$ » zum Reaktionsgemisch ist gleichfalls im Hinblick auf eine Steigerung der Ausbeute am gewünschten Endprodukt von Vorteil.

Zur Isolierung der erwünschten Zielprodukte bieten sich hier verschiedene Möglichkeiten an :

Ist als Endprodukt die jeweilige freie Säure erwünscht so wird das erhaltene Reaktionsprodukt durch Behandlung mit einem sauren Ionenaustauscherharz von Kationen befreit und anschließend eingeengt, wobei die erwartete Diphosphonsäure anfällt.

Andererseits lassen sich die erfindungsgemäßen Dihydroxydiphosphonsäuren auch in Form ihrer wasserlöslichen Salze, d. h. insbesondere der Alkalimetall-Ammonium- oder Alkanolaminsalze, vorzugsweise der Natrium- oder Kaliumsalze, isolieren.

So läßt sich beispielsweise das Natriumsalz — im Falle der Verwendung von Natriumnitrit — gewinnen, indem man den pH-Wert im Reaktionsprodukt mit Hilfe von verdünnter Natronlauge auf beispielsweise etwa 4.8 einstellt und anschließend mittels eines Lösungsmittels wie Aceton oder Methanol ausfällt.

Sofern andere Salze der Dihydroxydiphosphonsäure erwünscht sind, werden — vor der Neutralisation mit der entsprechenden Lauge — andere im Reaktionsgemisch vorliegenden Kationen, wie beispielsweise Natriumionen, unter Verwendung eines sauren Ionenaustauscherharzes zuvor entfernt.

Sofern sich im Reaktionsprodukt gegebenenfalls noch nicht umgesetzte Aminodiphosphonsäure befindet, was sich chromatographisch leicht nachweisen läßt, wird die klare Reaktionslösung — vor der Isolierung des Endproduktes — gleichfalls mit einem sauren Ionenaustauscherharz behandelt und anschließend eingeengt, wobei die schwerer lösliche Aminodiphosphonsäure aus der Lösung ausfällt und abgetrennt werden kann.

Die Reaktion primärer Alkylamine mit Salpetriger Säure unter Bildung der entsprechenden hydroxylgruppenhaltigen Verbindungen ist im Prinzip bekannt. Jedoch werden hierbei in der Regel unerwünschte Nebenprodukte, wie beispielsweise solche mit olefinischen Doppelbindungen, in erheblicher Menge

gebildet.

Überraschenderweise hat sich gezeigt, daß bei Durchführung der Reaktion in der beschriebenen Weise keine Verbindungen mit olefinischen Doppelbindungen und auch praktisch keine anderen Nebenprodukte auftreten, sondern vielmehr die erwünschten Dihydroxyalkandiphosphonsäuren.

Die neuen Dihydroxyalkandiphosphonsäuren beziehungsweise deren wasserlösliche Salze besitzen ein ausgezeichnetes Komplexiervermögen gegenüber zwei- und mehrwertigen Metallionen, wie beispielsweise gegenüber Ca, Mg, Cu, Fe, Cr und anderen, bei verschiedenen Temperaturen und pH-Werten.

Insbesondere zeigen sie ein besonders gutes Calciumbindevermögen, welches besser ist als dasjenige der Aminogruppen-enthaltenden analogen Verbindungen, wie sich in einfacher Weise durch den « Hampshire-Test » nachweisen läßt.

Demzufolge sind die neuen Dihydroxyalkandiphosphonsäuren beziehungsweise deren wasserlöslichen Salze geeignet zur Verwendung als Komplexier beziehungsweise Sequestriermittel.

Beispielhaft für die neuen Dihydroxyalkandiphosphonsäuren seien hier die folgenden Verbindungen genannt :

1,3-Dihydroxypropan-1,1-diphosphonsäure
1,4-Dihydroxybutan-1,1-diphosphonsäure
1,5-Dihydroxypentan-1,1-diphosphonsäure
1,6-Dihydroxyhexan-1,1-diphosphonsäure
1,11-Dihydroxyundecan-1,1-diphosphonsäure
1,3-Dihydroxy-3-phenylpropan-1,1-diphosphonsäure,

wobei der Phenylrest auch durch z. B. Halogen, Alkylreste, Hydroxylgruppen substituiert sein kann, sowie die entsprechenden wasserlöslichen Salze.

Im Sinne der Erfindung sind hiervon insbesondere Verbindungen gemäß der Formel I mit R = H und n = 1-4, vorzugsweise 1,3-Dihydroxypropan-1,1-diphosphonsäure und 1,6-Dihydroxyhexan-1,1-diphosphonsäure, beziehungsweise deren wasserlösliche Salze bevorzugt, da diese — verglichen mit den übrigen — ein wesentlich höheres Sequestriervermögen aufweisen.

Außer dem ausgezeicheten Komplexiervermögen zeichnen sich die neuen Diphosphonsäuren beziehungsweise deren wasserlösliche Salze durch eine starke Threshold-Aktivität aus, d. h. sie sind in der Lage, die Ausfällung schwerlöslicher Erdalkalimetallsalze auch in Impfmengen, das sind unterstöchiometrische Mengen, zu verhindern.

Die neuen Dihydroxyalkandiphosphonsäuren und deren wasserlösliche Salze sind als Komplexiermittel sehr vielseitig verwendbar : Beispielsweise können sie speziell für die Vorgänge der Wasserenthärtung Anwendung finden, wobei die vorstehend erwähnte Threshold-Wirkung eine wesentliche Rolle spielt. Es ist daher nicht notwendig, mit stöchiometrischen Mengen zu arbeiten, sondern man kann auch mit unterstöchiometrischen Mengen Calcitfällungen erheblich verzögern.

Sie sind auch als Korrosions- und Steinansatzverhütungsmittel für Kühlwässer, insbesondere in Kombination mit an sich bekannten Zusätzen, gut geeignet.

Die genannten Eigenschaften bewirken, daß die neuen Diphosphonsäuren beispielsweise auch für die Entkrustang von Geweben, in denen sich Erdalkalimetallsalze abgelagert haben, und zur Verminderung der Ascheanreicherung in Geweben Anwendung finden können. Schließlich kommen sie auch als Buildersubstanzen mit komplexierenden Eigenschaften in Wasch- und Reinigungsmitteln in Frage und können in Kombination mit bekannten anionenaktiven, kationenaktiven oder nichtionogenen Netzmitteln verwendet werden. Weiterhin können sie in Kombination mit Ätzalkalien, Alkalicarbonaten, silikaten, Phosphaten oder Boraten Anwendung finden.

Aufgrund des starken Komplexiervermögens der erfindungsgemäßen Säuren bzw. deren Salze, können diese auch mit Vorteil in Systemen eingesetzt werden, in denen Schwermetallionen, beispielsweise Kupferionen, unerwünschte Effekte auslösen. Beispielhaft seien hier genannt : Die Zersetzung von Perverbindungen in Bleichmitteln bzw. Verfärbungen oder Ranzigwerden von Fetten und Seifen.

Sie sind ferner geeignet für Reinigungsprozesse von starren Gegenständen wie insbesondere Metall oder Glas. Hierbei kommt insbesondere die Verwendung als Zusatz zu Flaschenspülmitteln in Betracht.

Die erfindungsgemäßen Produkte sind auch geeignet für pharmazeutische Zwecke, insbesondere zur Behandlung von Störungen des Calcium- beziehungsweise des Phosphatstoffwechsels und den damit verbundenen Erkrankungen. Weiterhin können die neuen Diphosphonsäuren und deren wasserlösliche Salze in kosmetischen Präparaten, wie insbesondere Zahnpasta, Mundwasser und ähnlichen Produkten, verwendet werden, da sie die Bildung von Zahnstein wesentlich reduzieren beziehungsweise inhibieren.

Schließlich sind die neuen Phosphonsäuren auch geeignet zur Herstellung von $99^{m}$-Technetium-Radiodiagnostika.

Der Anmeldungsgegenstand wird durch die nachstehenden Beispiele erläutert, ohne daß er hierauf beschränkt ist.

## Beispiel 1

0,1 Mol 3-Amino-1-hydroxypropan-1,1-diphosphonsäure wurden in 300 ml Wasser suspendiert und über eine Zeit von 12 Stunden 0,4 Mol 5 %ige Natriumnitritlösung zugetropft. Zur Entfernung nicht umgesetzter geringer Restmengen an Ausgangsverbindung wurde die klare Reaktionslösung mit einem

sauren Austauscherharz behandelt und dann eingeengt, so daß die schwerlösliche Aminodiphosphonsäure abgetrennt werden konnte.

Zur Isolierung des Endproduktes in Form des $Na_2$-Salzes wurde das Filtrat mit verdünnter Natronlauge auf pH 4,8 gestellt und das Dinatrium-1,3-dihydroxypropan-1,1-diphosphonat mit Aceton ausgefällt. Das Salz wurde bei 50 °C im Vakuum getrocknet.

Ausbeute : 85 %.

| Elementaranalyse : | P | : | C | : | Na | |
|---|---|---|---|---|---|---|
| | 2.00 | : | 3.14 | : | 2.08 | gefunden |
| | ( 2 | : | 3 | : | 2 ) | errechnet |

Beispiel 2

In eine Suspension von 0,1 Mol 3-Amino-3-phenyl-1-hydroxypropan-1,1-diphosphonsäure in 250 ml Wasser wurden nach Erwärmen auf 50 °C 0,4 Mol Natriumnitritlösung innerhalb von 15 Stunden getropft.

Anschließend wurde die Reaktionslösung durch Behandlung mit saurem Austauscherharz von $Na^+$-Ionen befreit und eingeengt. Aus der eingeengten Lösung wurden 62 % 3-Phenyl-1,3-dihydroxypropan-1,1-diphosphonsäure gewonnen.

Smp. 132 °C.

| %C | | %H | | %P | | |
|---|---|---|---|---|---|---|
| | 34.6 | | 5.01 | | 19.9 | gefunden |
| | (34.62) | | (4.49) | | (19.87) | errechnet |

Beispiel 3

0,1 Mol 6-Amino-1-hydroxyhexan-1,1-diphosphonsäure wurden in gleicher Weise wie in Beispiel 1 behandelt und zur Vervollständigung der Desaminierung noch 10 Stunden nachgerührt. Die weitere Aufarbeitung analog Beispiel 1 führte zur Abtrennung des $Na_2$-Salzes der 1,6-Dihydroxyhexan-1,1-diphosphonsäure in über 80 %iger Ausbeute.

| Elementaranalyse : | P | : | C | : | Na | |
|---|---|---|---|---|---|---|
| | 2.00 | : | 6.18 | : | 2.01 | gefunden |
| | (2 | : | 6 | : | 2 ) | errechnet |

Beispiel 4

Analog Beispiel 1 wurde eine Suspension von 0,1 Mol 11-Amino-1-hydroxyundecan-1,1-diphosphonsäure mit 0,4 Mol einer Na-Nitritlösung behandelt und mehrere Stunden bei 50 °C nachgerührt. Dann wurden ggf. nicht gelöste Anteile an Ausgangsverbindung abfiltiert und das Filtrat mit konz. Salzsäure auf pH 1 gestellt, wobei ein Öl abgeschieden wurde, das nach Trocknen im Vakuum als 1,11-Dihydroxyundecan-1,1-diphosphonsäure identifiziert wurde ; Ausbeute : 41 %.

Smp. 112 °C

| %C | | %H | | %P | | |
|---|---|---|---|---|---|---|
| | 38.4 | | 7.44 | | 17.6 | gefunden |
| | (37.93) | | (7.47) | | (17.82) | errechnet |

Beispiel 5

Hampshire-Test zur Bestimmung des Calciumbindevermögens bei pH 11.

Circa 1 000 mg der unten angegebenen Diphosphonsäuren wurden in 80 bis 90 ml Wasser gelöst, mit Natronlauge auf pH 11 gestellt, 10 ml 2 %ige Sodalösung zugesetzt und eine Calciumsalzlösung (36,8 g $CaCl_2 \times 2H_2O$/1) langsam bis zur bleibenden Trübung zugetropft.

Die erhaltenen Ergebnisse enthält die folgende Tabelle :

| Substanz | mg $CaCO_3$ pro g Säure |
|---|---|
| 1,3-Dihydroxypropan-1,1-diphosphonsäure | 632 |

Vergleich : 3-Amino-1-hydroxypropan-1,1-diphosphonsäure 472
1,6-Dihydroxyhexan-1,1-diphosphonsäure 940
Vergleich : 6-Amino-1-hydroxyhexan-1,1-diphosphonsäure 402

Die Ergebnisse zeigen, daß mit Hilfe der erfindungsgemäßen Diphosphonsäuren eine wesentlich größere Menge an $CaCO_3$ durch Komplexierung vollständig in Lösung gehalten wird, als von der gleichen Menge der jeweiligen Vergleichssubstanz :

Beispiel 6

Die Threshold-Aktivität der Dihydroxyalkan-diphosphonsäuren ist in der nachfolgenden Tabelle angegeben. Die Werte zeigen, daß noch bis zu einer Impfmenge von 1 ppm eine Calciumsulfat-Inhibierung erfolgt.

Zur Bestimmung des Threshold-Vermögens wurden die in der Tabelle angegebenen Impfmengen mit 50 ml einer Calciumsalzlösung (11,1 g $CaCl_2 \cdot 2H_2O$/1 und 7,5 g NaCl/1) mit 50 ml einer Sulfatlösung (10,66 g $Na_2SO_4$/1 und 7,5 g $Na_2$ Cl/1) versetzt und die Mischungen 72 Stunden bei 70 °C stehen gelassen.

Anschließend wurde der in Lösung verbliebene Anteil an Calciumionen bestimmt.

| Dihydroxyalkan- | Gips-Inhibierung in mg/l | | | | |
|---|---|---|---|---|---|
| diphosphonsäure | 1 ppm | 3 ppm | 5 ppm | 10 ppm | 20 ppm |
| 1 | 4814 | 5005 | 4910 | 4978 | 4964 |
| 2 | 4624 | 5114 | 5222 | 5236 | 5209 |
| 3 | 3713 | 3822 | 3917 | 4570 | 5127 |

Blindprobe ohne Inhibitor : gef. 3522 ber 5134

1 = 1,3-Dihydroxypropan-1,1-diphosphonsäure
2 = 1,6-Dihydroxyhexan-1,1-diphosphonsäure
3 = 1,3-Dihydroxy-3-phenylpropan-1,1-diphosphonsäure

Beispiel 7

Bei Verwendung der erfindungsgemäßen Dihydroxyalkandiphosphonsäuren oder ihrer pharmazeutisch akzeptablen Salze in Mund- und Zahnpflegemitteln wird die Bildung von Zahnstein wesenlich reduziert beziehungsweise inhibiert. Der pH-Wert der Mundwässer oder Zahnpasten kann sich zwischen den Grenzen 5 bis 9 bewegen.

Als Grundrezepturen für Zahnpasten sind beispielsweise folgende Formulierungen geeignet :

a.

| | |
|---|---|
| Glycerin | 60,0 Gew.-Teile |
| Wasser | 13,5 Gew.-Teile |
| Natriumcarboxymethylcellulose | 0,6 Gew.-Teile |
| Kieselsäurexerogel | 20,0 Gew.-Teile |
| Natriumlaurylsulfat | 2,0 Gew.-Teile |
| Etherische Öle | 1,0 Gew.-Teile |
| Süßstoff | 0,4 Gew.-Teile |
| 1,3-Dihydroxypropan-1,1-diphosphonsäure | 2,5 Gew.-Teile |

b.

| | |
|---|---|
| Glycerin | 30,0 Gew.-Teile |
| Wasser | 18,5 Gew.-Teile |
| Natriumcarboxymethylcellulose | 1,0 Gew.-Teile |
| Aluminiumhydroxyid | 44,0 Gew.-Teile |
| Natriumlaurylsulfat | 1,0 Gew.-Teile |
| Kieselsäure, pyrogen | 1,5 Gew.-Teile |
| Etherische Öle | 1,5 Gew.-Teile |
| Süßstoff | 0,5 Gew.-Teile |
| 1,6-Dihydroxyhexan-1,1-diphosphonsäure | 2,0 Gew.-Teile |

Als Grundrezeptur für Mundwässer ist beispielsweise folgende Kombination geeignet :

| | |
|---|---|
| Ethylalkohol | 19,5 Gew.-Teile |
| Glycerin | 7,5 Gew.-Teile |
| Wasser | 70,0 Gew.-Teile |
| Etherische Öle | 0,2 Gew.-Teile |
| Natriumlaurylsulfat | 0,1 Gew.-Teile |
| Antiseptikum (Chlorthymol) | 0,1 Gew.-Teile |
| Süßstoff | 0,1 Gew.-Teile |
| 1,3-Dihydroxypropan-1,1-diphosphonsäure | 2,5 Gew.-Teile |

Durch den regelmäßigen Gebrauch der Zahnpasten und/oder Mundwässer mit einem Gehalt an Natriumsalzen der erfindungsgemäßen Diphosphonsäuren läßt sich die Bildung von Zahnstein wesentlich verringern und die Ausbildung von harten, kompakten Zahnbelägen weitgehend verhindern.

**Patentansprüche**

1. α,ω-Dihydroxyalkan-α,α-diphosphonsäuren der allgemeinen Formel (I)

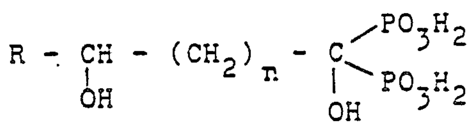
(I)

worin R ein Wasserstoffatom, einen Phenylrest oder einen durch Halogen, Alkylreste oder Hydroxylgruppen substituierten Phenylrest und n eine ganze Zahl im Bereich von 1 bis 9 bedeuten, sowie deren wasserlösliche Salze.

2. Verbindungen gemäß Anspruch 1 dadurch gekennzeichnet, daß R ein Wasserstoffatom und n eine ganze Zahl im Bereich von 1 bis 4 bedeuten.

3. 1,3-Dihydroxypropan-1,1-diphosphonsäure und deren wasserlösliche Salze.

4. 1,6-Dihydroxyhexan-1,1-diphosphonsäure und deren wasserlösliche Salze.

5. Verwendung von Verbindungen gemäß Anspruch 1 bis 4 als Komplexiermittel gegenüber zwei- und mehrwertigen Metallionen.

**Claims**

1. α,ω-Dihydroxyalkane-α,α-diphosphonic acids having the following general formula

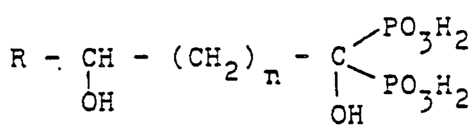
(I)

in which R is a hydrogen atom, a phenyl radical or a halogenalkyl- or hydroxyl-substituted phenyl radical and n is an integer in the range from 1 to 9, and water-soluble salts thereof.

2. Compounds as claimed in claim 1, characterized in that R is a hydrogen atom and n is an integer in the range from 1 to 4.

3. 1,3-Dihydroxypropane-1,1-diphosphonic acid and water-soluble salts thereof.

4. 1,6-Dihydroxyhexane-1,1-diphosphonic acid and water-soluble salts thereof.

5. The use of the compounds claimed in claim 1 to 4 as complexing agents for divalent and polyvalent metal ions.

**Revendications**

1. Acides α,ω-dihydroxy-alcane-α,α-diphosphoniques de formule générale (I) :

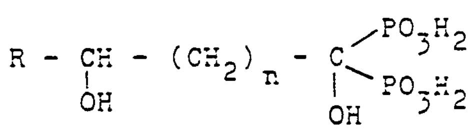
(I)

dans laquelle R représente un atome d'hydrogène, un reste phényle ou un reste phényle substitué par un halogène, des restes alcoyle ou des groupes hydroxyle et n est un nombre entier compris entre 1 et 9, ainsi que leurs sels solubles dans l'eau.

2. Composés selon la revendication 1, caractérisés par le fait que R représente un atome d'hydrogène et n un nombre entier compris entre 1 et 4.

3. L'acide 1,3-dihydroxy-propane-1,1-diphosphonique et ses sels solubles dans l'eau.

4. L'acide 1,6-dihydroxy-hexane-1,1-diphosphonique et ses sels solubles dans l'eau.

5. Utilisation des composés selon les revendications 1 à 4 comme agents complexants vis-à-vis des ions métalliques divalents et multivalents.